# EUROPEAN PATENT APPLICATION

(11) **EP 1 148 100 A2**
(43) Date of publication of application: **24.10.2001**
(21) Application number: 01303493.9
(22) Date of filing: 17.04.2001
(51) Int. Cl.: C08L 83/12, C08G 77/20, C08G 77/46, C11D 3/50, A61K 7/46

(54) **Fragrance containing organopolysiloxane composition and method of preparation**

(30) Priority: 17.04.2000 JP 2000114703
(71) Applicant: Dow Corning Toray Silicone Co., Ltd., Tokyo (JP)
(72) Inventor: Kondo, Hidetoshi, Ichihara-shi, Chiba Prefecture (JP); Takahashi, Masahiro, Ichihara-shi, Chiba Prefecture (JP)
(74) Representative: Kyle, Diana

(57) **Abstract**

An organopolysiloxane composition comprising 5 to 99.9 wt% of a crosslinked product of organopolysiloxane which is liquid or gel-like at normal temperature, the crosslinked product containing silicon-bonded polyoxyalkylene groups described by general formula ―R¹(OR²)ₘOR³, where R¹ and R² are independently selected alkylene groups, R³ is selected from the group consisting of a hydrogen atom, alkyl groups, acyl groups, and isocyan group, and *m* is a positive integer, and (B) 95 to 0.1 wt% of a fragrance material. The composition has superior fragrance retention capability when use to impart fragrance to other compositions such as hair conditioners, shampoos, and cosmetics.

## Description

The present invention relates to an organopolysiloxane composition containing a fragrance material and to a method for preparing the same, and more specifically relates to an organopolysiloxane composition possessing a superior fragrance retention capability and compounding stability, and to a method for preparing the same.

An organopolysiloxane composition comprising a cured product of a room temperature curable type organopolysiloxane composition and a fragrance material (see Japanese Patent Application Laying Open No. Sho 61-13961), an organopolysiloxane composition comprising silicone rubber particles, silicone oil, and a fragrance material (see Japanese Application Laying Open No. Hei 10-036228), and an organopolysiloxane composition comprising silicone oil and a fragrance material (see Japanese Application Laying Open 11-114042) are known as organopolysiloxane compositions containing fragrance materials.

The problem with these organopolysiloxane compositions, however, consisted in their insufficient fragrance retention. For example, when added to cosmetic materials, detergents, lustering agents, surface finishing agents, fiber treating agents, and coating materials they do not retain a fragrance for an extended time.

It is an object of the present invention to provide an organopolysiloxane composition which is superior in fragrance retention and exhibits excellent compounding stability when used as an additive for cosmetic materials, detergents, lustering agents, surface finishing agents, fiber treating agents, coating materials, etc., and furthermore which can impart excellent skin feel, finish properties, surface smoothness characteristics, and surface-protecting properties to the resultant compounds, as well as to provide a method for preparing the same.

The present invention relates to an organopolysiloxane composition comprising (A) 5 to 99.9 wt% of a crosslinked product of organopolysiloxane which is liquid or gel-like at normal temperature, the crosslinked product containing silicon-bonded polyoxyalkylene groups described by general formula―R¹(OR²)ₘOR³ , where R¹ and R² are independently selected alkylene groups, R³ is selected from the group consisting of a hydrogen atom, alkyl groups, acyl groups, and isocyan group, and m is a positive integer, and (B) 95 to 0.1 wt% of a fragrance material; and to the above-described organopolysiloxane composition emulsified in water, and to a method for preparing the same.

The present invention relates to an organopolysiloxane composition comprising (A) 5 to 99.9 wt% of a crosslinked product of organopolysiloxane which is liquid or gel-like at normal temperature, the crosslinked product containing silicon-bonded polyoxyalkylene groups described by general formula―R¹(OR²)ₘOR³, where R¹ and R² are independently selected alkylene groups, R³ is selected from the group consisting of a hydrogen atom, alkyl groups, acyl groups, and isocyan group, and *m* is a positive integer, and
(B) 95 to 0.1 wt% of a fragrance material; and to the above-described organopolysiloxane composition emulsified in water, and to a method for preparing the same.

First of all, a detailed explanation will be provided regarding the organopolysiloxane composition of the present invention. The crosslinked product of organopolysiloxane of component (A) used in the present composition is characterized in that it is liquid or gel-like at normal temperature and in that polyoxyalkylene groups represented by the general formula: ―R¹(OR²)ₘOR³ are bonded to the silicon atoms of which the crosslinked product is composed. In the above formula, R¹ and R² are the same or different alkylene groups exemplified by ethylene, propylene, butylene, isobutylene, pentamethylene, octamethylene, decamethylene, dodecamethylene, and cyclohexylene. Among these alkylene groups, ethylene, propylene, and butylene are preferable. R³ is a group selected from the group consisting of a hydrogen atom, alkyl groups, acyl groups, and an isocyan group; where the alkyl groups are exemplified by methyl, ethyl, propyl, butyl, isobutyl, pentyl, octyl, decyl, and dodecyl, and the acyl groups are exemplified by formyl, acetyl, propionyl, butylyl, isobutylyl, valeryl, oxalyl, malonyl, succinyl, and glutaryl. The subscript *m* is a positive integer, preferably, in the range of from 1 to 100, and, even more preferably, in the range of from 20 to 80. Polyoxyalkylenes described by general formula―R¹(OC₂H₄)ₚ(OC₃H₆)_{q}OR³ are preferable as such polyoxyalkylene groups. In the formula, R¹ and R³ are the same as above. The subscript *p* is 0 or a positive integer, preferably in the range of from 0 to 20. The subscript *q* is a positive integer, preferably in the range of 20 to 80. However, it is necessary that *p* should be smaller than *q* at all times. In addition, when *p* is a positive integer, the bond between oxyethylene and oxypropylene may be a block copolymer-type bond or a random copolymer-type bond. Specific examples of the polyoxyalkylene groups are described by the following formulas:

―(CH₂)₃(OC₂H₄)₁₀(OC₃H₆)₅₀OH

―(CH₂)₃(OC₃H₆)₅₀OH

―(CH₂)₃(OC₃H₆)₃₀OCH₃

―(CH₂)₃(OC₂H₄)₅(OC₃H₆)₃₀OCH₃

―(CH₂)₂(OC₂H₄)₅(OC₃H₆)₅₀OH

―(CH₂)₂(OC₃H₆)₂₀OCOCH₃

In addition, in the crosslinked product comprising component (A), silicon-bonded groups other than the above-mentioned polyoxyalkylene groups are exemplified by substituted or unsubstituted monovalent hydrocarbon groups, such as methyl, ethyl, propyl, butyl, pentyl, hexyl, heptyl, octyl, nonyl, decyl, hexadecyl, heptadecyl, octadecyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these, a small amount of methoxy, ethoxy, propoxy, and other alkoxy groups and hydroxyl groups may be present.

The term "liquid," as used in the present invention, indicates a substance with a viscosity in the range of from 1 mPa·s to 10,000,000 mPa·s at normal temperature, with a viscosity in the range of from 100,000 mPa·s to 10,000,000 mPa·s being particularly preferable. In addition, the term "gel-like" indicates a property, whereby a substance, albeit devoid of self-flowing capacity, undergoes an irreversible deformation when an external force is applied thereto.

A crosslinked product obtained by crosslinking an addition reaction-curable organopolysiloxane composition is preferable as the crosslinked product of organopolysiloxane of component (A). Specifically suggested is a crosslinked product obtained via a hydrosilation reaction between the below described component (a), component (b), and component (c), with the crosslinked product of a crosslinkable organopolysiloxane composition comprising the below-described components (a) to (d) particularly recommended. In addition, although crosslinking is conducted at room temperature, if necessary, it may be carried out under heating.

Component (a) is an organopolysiloxane containing silicon-bonded hydrogen atoms. Component (a) is crosslinked via a hydrosilation reaction with component (b) and component (c). In component (a) silicon-bonded groups other than hydrogen atoms are exemplified by substituted or unsubstituted monovalent hydrocarbon groups, such as methyl, ethyl, propyl, butyl, pentyl, hexyl, heptyl, octyl, nonyl, decyl, hexadecyl, heptadecyl, octadecyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these groups, a small amount of methoxy, ethoxy, propoxy, and other alkoxy groups and hydroxyl groups may be present. However, component (a) does not contain polyoxyalkylene groups.

There are no limitations concerning the structure of component (a) and suggested structures include, for example, linear, partially branched linear, branched, or ring structures, with linear or partially branched linear structures being preferable. There are no limitations concerning the viscosity of component (a) at 25°C; however, preferably it should be in the range of from 1 mPa·s to 100,000 mPa·s and even more preferably in the range of from 1 mPa·s to 10,000 mPa·s. In addition to dimethylpolysiloxane having both terminal ends of the molecular chain blocked by dimethylhydrogensiloxy groups, copolymer of methylhydrogensiloxane and dimethylsiloxane having both terminal ends of the molecular chain blocked by dimethylhydrogensiloxy groups, and copolymer of methylhydrogensiloxane and dimethylsiloxane having both terminal ends of the molecular chain blocked by trimethylsiloxy groups, component (a) is exemplified by organopolysiloxanes obtained by substituting phenyl, ethyl, lauryl, stearyl, 3,3,3-trifluoropropyl, etc. for some of the methyl groups in the above-mentioned siloxanes.

Component (b) is a characteristic component of the present invention used for improving the retention of the fragrance material in the present composition. Component (b) is (b-1) a polyoxyalkylene described by formula R⁴(OR²)ₘOR³ or (b-2) an organopolysiloxane having silicon-bonded polyoxyalkylene groups represented by the general formula: ―R¹(OR²)ₘOR³, and alkenyl groups or hydrogen atoms. Although typically only one component among components (b-1) and (b-2) is used, both components can be mixed and used simultaneously. In the polyoxyalkylene of the above-described component (b-1), R², R³, and *m* are the same as above. R⁴ is an alkenyl group exemplified by vinyl, allyl, butenyl, pentenyl, and hexenyl. A polyoxyalkylene represented by the general formula R⁴(OC₂H₄)ₚ(OC₃H₆)_{q}OR³ is preferable as component (b-1). In the formula, *p* and *q* are the same as above. Specifically, oxyalkylene compounds represented by the following formulas are suggested as examples.

CH₂=CHCH₂―(OC₂H₄)₁₀(OC₃H₆)₅₀OH

CH₂=CHCH₂―(OC₃H₆)₅₀OH

CH₂=CHCH₂―(OC₃H₆)₃₀OCH₃

CH₂=CHCH₂―(OC₂H₄)₅(OC₃H₆)₃₀OCH₃

CH₂=CH―(OC₂H₄)₅(OC₃H₆)₅₀OH

CH₂=CH―(OC₃H₆)₂₀OCOCH₃

In the organopolysiloxane of the above-mentioned component (b-2), R¹, R², R³, and *m* are the same as above. This organopolysiloxane is characterized by having one or more of the above-mentioned polyoxyalkylene groups and alkenyl groups or silicon-bonded hydrogen atoms in one molecule. The alkenyl groups are exemplified by vinyl, allyl, butenyl, pentenyl, and hexenyl. In addition to these groups, silicon-bonded groups are exemplified by substituted or unsubstituted monovalent hydrocarbon groups, such as methyl, ethyl, propyl, butyl, pentyl, hexyl, heptyl, octyl, nonyl, decyl, hexadecyl, heptadecyl, octadecyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these groups, a small amount of methoxy, ethoxy, propoxy, and other alkoxy groups and hydroxyl groups may be present. There are no limitations concerning structure of (b-2) and suggested structures include, for example, linear, partially branched linear, branched, or ring structures, with linear or partially branched linear structures being preferable. The viscosity of (b-2) at 25°C is preferably in the range of from 1 mPa·s to 500,000 mPa·s, and even more preferably in the range of from 1 mPa·s to 50,000 mPa·s. The organopolysiloxane of component (b-2) is exemplified by siloxanes described by the following formulas

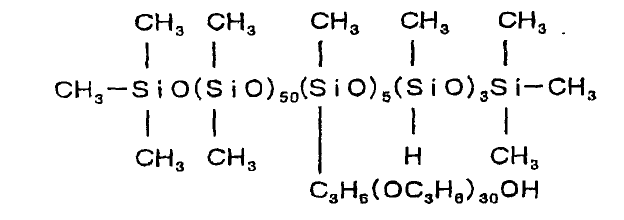

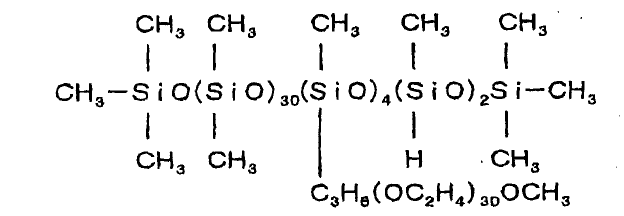

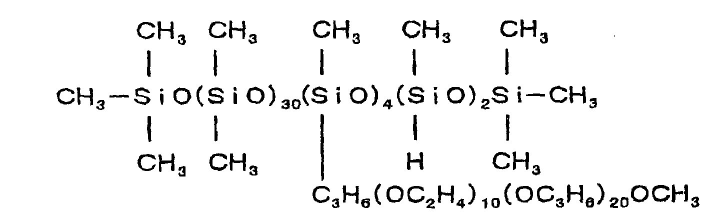

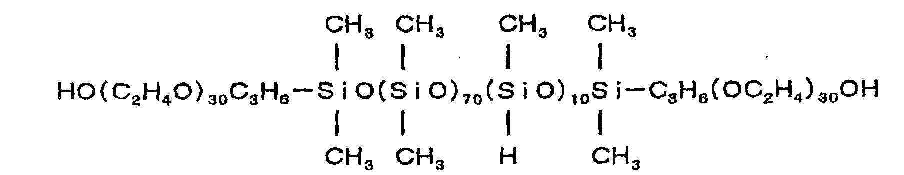

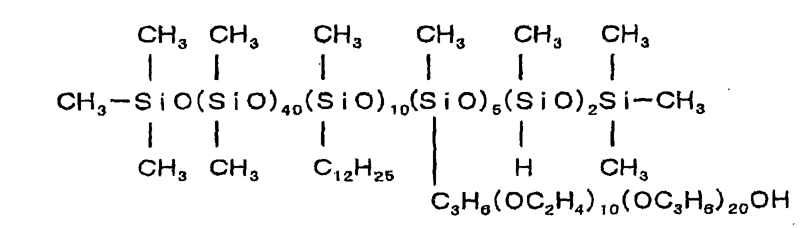

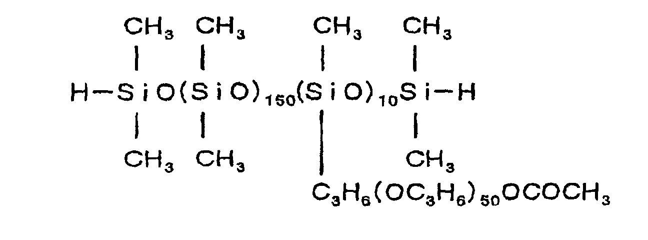

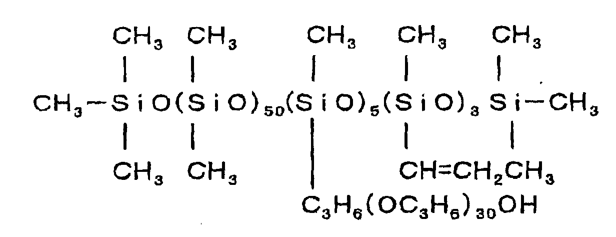

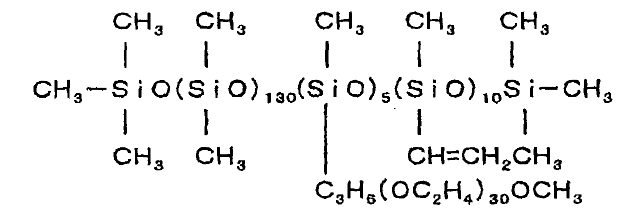

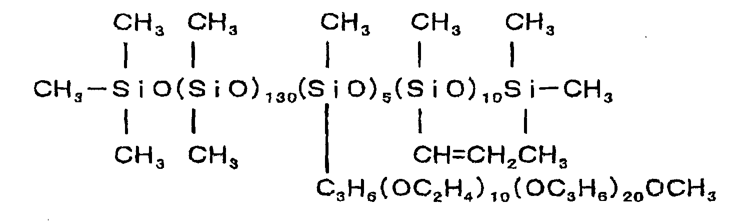

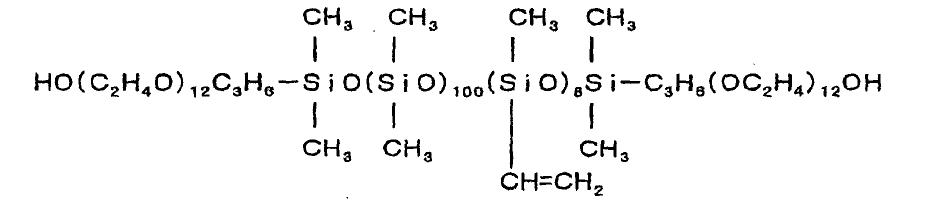

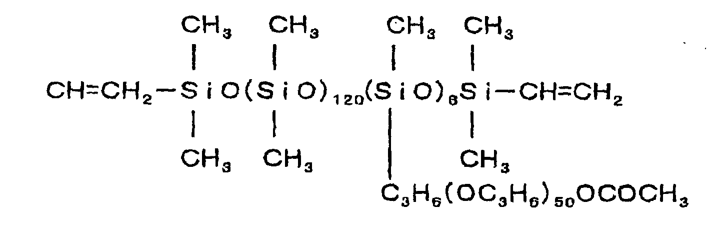

The amount of added component (b) is in the range of 0.1 to 100 parts by weight, preferably in the range of 0.5 to 80 parts by weight, and especially preferably in the range of 1 to 40 parts by weight per 1 part by weight of component (a). This is due to the fact that when the amount of added component (b) is less than 0.1 parts by weight, the fragrance retention capability of the present composition tends to decrease, and when it exceeds 100 parts by weight the skin feel, finish properties, surface smoothness characteristics, surface-protecting properties, and other characteristics of the resultant compounds tend to deteriorate when the present composition is added to, for example, cosmetic materials, detergents, lustering agents, surface finishing agents, fiber treating agents, and coating materials.

The organopolysiloxane of component (c) is an organopolysiloxane having at least two silicon-bonded alkenyl groups in each molecule. The alkenyl groups are exemplified by vinyl, allyl, butenyl, pentenyl, and hexenyl. In this organopolysiloxane, silicon-bonded groups other than alkenyl groups are exemplified by substituted or unsubstituted monovalent hydrocarbon groups, such as methyl, ethyl, propyl, butyl, pentyl, hexyl, heptyl, octyl, nonyl, decyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these groups, a small amount of methoxy, ethoxy, propoxy, and other alkoxy groups and hydroxyl groups may be present. Component (c) does not contain polyoxyalkylene groups. Suggested structures for component (c) include linear, partially branched linear, branched, or ring structures, with linear or partially branched linear structures being especially preferable. There are no limitations concerning the viscosity of component (c) at 25°C; preferably the viscosity should be in the range of from 10 mPa·s to 100,000 mPa·s and even more preferably in the range of from 10 mPa·s to 10,000 mPa·s. In addition to dimethylpolysiloxane having both terminal ends of the molecular chain blocked by dimethylvinylsiloxy groups, copolymer of methylvinylsiloxane and dimethylsiloxane having both terminal ends of the molecular chain blocked by dimethylvinylsiloxy groups, and copolymer of methylvinylsiloxane and dimethylsiloxane having both terminal ends of the molecular chain blocked by trimethylsiloxy groups; component (c) is exemplified by organopolysiloxanes obtained by substituting, for example, phenyl, ethyl, lauryl, stearyl, and 3,3,3-trifluoropropyl groups for some of the methyl groups in the above-mentioned siloxanes. The amount of added component (c) is in the range of 0.01 to 100 parts by weight, preferably in the range of 0.1 to 70 parts by weight, and especially preferably in the range of 1 to 50 parts by weight per 1 part by weight of component (a). This is due to the fact that when the amount of added component (c) is less than 0.01 parts by weight, the skin feel, finish properties, surface smoothness characteristics, surface-protecting properties, and other characteristics of the resultant compounds tend to deteriorate when the present composition is added to cosmetic materials, detergents, lustering agents, surface finishing agents, fiber treating agents, coating materials, etc.; and on the other hand when it exceeds 100 parts by weight the fragrance retention capability of the present composition tends to decrease.

The hydrosilation reaction catalyst of component (d) is used for promoting a hydrosilation reaction between the above-described components (a) to (c). Platinum catalysts, rhodium catalysts, and palladium catalysts are suggested as such catalysts. Among these catalyst, platinum catalysts are preferred, and specifically suggested catalysts include chloroplatinic acid, alcohol solutions of chloroplatinic acid, olefin complexes of platinum, alkenylsiloxane complexes of platinum, carbonyl complexes of platinum, platinum black, platinum catalysts supported on silica, as well as their mixtures. The amount of added component (d) is a catalytic amount. When a platinum catalyst is used, the amount is preferably such that the concentration of platinum metal is within the range of 0.01 to 1,000 parts by weight per 1,000,000 parts by weight of the total of component (a) to component (c). This is due to the fact that when the platinum metal is less than 0.01 parts by weight, the hydrosilation reaction does not proceed sufficiently to completion, and when an amount exceeding 1,000 parts by weight is added the catalytic effect is not increased.

Natural fragrance materials, synthetic fragrance materials, or mixed fragrance materials are suggested as the fragrance material of component (B) used in the present composition. With account taken of their solubility in the crosslinked product of organopolysiloxane, the fragrance materials are specifically exemplified by hexanol, heptanol, octanol, nonanol, decanol, *cis*-3-hexenol, and other aliphatic alcohols; hexanal, heptanal, octanal, nonanal, decanal, 10-undecenal, and other aliphatic aldehydes; 2-octanone, methylheptenone, and other aliphatic ketones; isopentyl acetate, *cis*-3-hexenyl acetate, allyl cyclohexylpropionate, and other aliphatic esters; isoparaffin, and other aliphatic hydrocarbons; D-limonene, P-cymene and other terpene-series hydrocarbons; linalool, terpineol, citronellol, and other terpene-series alcohols; citral, citronellal, and other terpene-series aldehydes; camphor, L-carbone, menthone, and other terpene-series ketones; geranyl acetate, linalyl propionate, citronellyl isobutyrate, and other terpene-series esters; rose oxides, linalool oxides, and other terpene-series ethers; lemon oil, orange oil, lime oil, and other citrus essential oils; lavender oil, rosemary oil, peppermint oil, and other herbal essential oils; rose oil, neroli oil, and other floral essential oils; cyclopentadecanolide, and other synthetic musk fragrances. Such fragrance materials can be used singly or as a mixture of two or more materials. In addition, these fragrance materials are typically liquid at normal temperature.

The proportion, in which the above-described (A) crosslinked product of organopolysiloxane and (B) fragrance material are compounded, is in the range of 5 to 99.9 : 95 to 0.1 wt%, preferably, in the range of from 25 to 99 : 75 to 1 wt%, and even more preferably in the range of 40 to 99 : 60 to 1 wt%. This is due to the fact that when the content of the fragrance material is less than 0.1 wt%, the fragrance cannot be imparted when the present composition is added to cosmetic materials, detergents, lustering agents, surface finishing agents, fiber treating agents, coating materials, and the like, and when it exceeds 95 wt% the fragrance retention capability of the present composition tends to decrease.

Although there are no limitations concerning the form of the present composition, an emulsion produced by emulsification in water is preferable. It is desirable to use a surface active agent in the preparation of the emulsion in order to improve the emulsion stability of the aforementioned crosslinkable organopolysiloxane composition. The surface active agents to be used are exemplified by anionic surface active agents, such as hexylbenzenesulfonic acid, octylbenzenesulfonic acid, decylbenzylsulfonic acid, dodecylbenzenesulfonic acid, cetylbenzenesulfonic acid, myristylbenzenesulfonic acid and their salts; cationic surface active agents, such as octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow trimethylammonium hydroxide, coconut oil trimethylammonium hydroxide; nonionic surface active agents of the polyester series, as well as ethylene oxide adduct of diethylene glycol trimethyl nonanol, polypropylene glycol, polyethylene glycol, polyoxyalkylene sorbitan ester, polyoxyalkylene alkyl ester, polyoxyalkylene alkyl phenol, and polyoxyalkylene alkyl ether. Such surface active agents can be used singly or as a mixture of two or more agents. The use of nonionic surface active agents for emulsification is particularly preferable when using the present composition as an additive for cosmetic products. There are no limitations concerning the amount of compounded surface active agent, but preferably it is in the range of 0.01 to 50 parts by weight and even more preferably in the range of 0.1 to 20 parts by weight per 100 parts by weight of the present composition. This is due to the fact that when it is less than 0.01 parts by weight, the stability of the emulsion decreases and when it exceeds 50 parts by weight it adversely affects the characteristics of the resultant compounds when the composition is added to, for example, cosmetic materials, detergents, lustering agents, surface finishing agents, fiber treating agents, and coating materials. In addition, although there are no limitations concerning the amount of added water, preferably it is in the range of 10 to 1,000 parts by weight per 100 parts by weight of the present composition. This is due to the fact that when the water is less than 10 parts by weight, the stability of the emulsion decreases, and when it exceeds 1,000 parts by weight sufficient fragrant properties cannot be imparted to, for example, cosmetic materials, detergents, lustering agents, surface finishing agents, fiber treating agents, and coating materials.

Next, a detailed description will be provided regarding the method of preparing the present organopolysiloxane composition. Such methods include a method where 5 to 99.9 wt% of a crosslinkable organopolysiloxane composition comprising the above-described components (a) to (d) and 95 to 0.1 wt% of a fragrance material are subjected to crosslinking in an emulsified state in water and a method where the fragrance material is added after crosslinking a crosslinkable organopolysiloxane composition comprising the above-described components (a) to (d) in an emulsified state in water.

In the former method, a process in which the above-described components (a) to (c) and the fragrance material are emulsified in advance and then component (d) is added thereto is preferable. It is preferable to use colloid mills, homogenizers, homomixers, and other emulsifying equipment, as well as high-shear agitators used for high-viscosity liquids. At such time, it is preferable to admix the above-described surface active agents, the use of nonionic surface active agent being especially preferable. Although the crosslinking reaction after emulsification proceeds even at room temperature, if necessary the emulsion may be heated.

In addition, in the latter method, a process in which an emulsion is prepared by emulsifying the above-described components (a) to (c) in water in advance, and then component (d) is added to the emulsion is preferable. It is preferable to use colloid mills, homogenizers, homomixers, and other emulsifying equipment, as well as high-shear agitators used for high-viscosity liquids. At such time, it is preferable to admix the above-described surface active agents, the use of nonionic surface active agent being especially preferable. Although the crosslinking reaction after emulsification proceeds even at room temperature, if necessary the emulsion may be heated. The fragrance material is added after preparing an aqueous emulsion of the crosslinked product of organopolysiloxane in this manner, with the mixing ratio of the crosslinked product of organopolysiloxane and the fragrance material being in the range of 5 to 99.9 : 95 to 0.1 wt%.

Because the present organopolysiloxane composition is superior in fragrance retention capability, when it is used as an additive for various cosmetic materials, detergents, lustering agents, surface finishing agents, fiber treating agents, coating materials, etc., the products are characterized by retaining the fragrance over an extended period of time. Furthermore, the present composition is superior in compounding stability with respect to various materials and can impart excellent skin feel, finishing properties, surface smoothness characteristics, surface-protective properties, and other characteristics to the resultant products. In addition although the preparative method of the present invention yields an aqueous emulsion of the present composition, if necessary, water can be removed.

### Application Examples

Hereinbelow, the present invention is explained in detail by referring to application examples. In the application examples, the term "viscosity" refers to a value obtained at 25°C. A liquid mixed fragrance material obtained by blending limonene, cyclohexylaldehyde, and allyl heptate in a proportion of 1:1:1 was used as the fragrance material. In addition, the fragrance retention properties, compounding stability, and flexibility and smoothness were measured in accordance with the measurement methods described below.
○ Fragrance retention properties
   A bundle of hair with a length of 15 cm and a weight of 15g was washed in an aqueous solution of sodium polyoxyethylene alkyl sulfate, rinsed, and then a test sample in the amount of 1g was applied thereto. The hair was allowed to stand indoors at a temperature of 20°C and a humidity of 40% and the presence of the scent 1 day, 2 days, and 3 days later was evaluated in the following manner.
   A: A distinct scent could be discerned.
   B: A faint scent could be discerned.
   C: No scent at all could be discerned.
○ Compounding stability
   After allowing the sample to stand for 1 day at 50°C, its appearance was visually evaluated in the following manner.
   A: Uniform.
   B: Slight separation detected.
   C: Completely separated.
○ Flexibility & Smoothness
   A bundle of hair with a length of 15 cm and a weight of 15g was washed in an aqueous solution of sodium polyoxyethylene alkyl sulfate, rinsed, and then a test sample in the amount of 1g was applied thereto. The hair was allowed to dry indoors and the flexibility and smoothness of the hair was evaluated by tactile sensation in accordance with the following evaluation criteria.
   A: Excellent.
   B: Good.
   C: Fair.
   D: Poor.

Application Example 1. A mixture was prepared comprising 1.7 parts by weight of a copolymer (content of silicon-bonded hydrogen atoms: 0.8 wt%) of dimethylsiloxane and methylhydrogensiloxane having both terminal ends of the molecular chain blocked by trimethylsiloxy groups, which had a viscosity of 15 mPa·s, 25.3 parts by weight of polyoxypropylene described by formula CH₂=CHCH₂(OC₃H₆)₅₀OH, which had one terminal end of the molecular chain blocked by an allyl group, 20 parts by weight of dimethylpolysiloxane (content of vinyl groups: 0.5 wt%) having both terminal ends of the molecular chain blocked by dimethylvinylsiloxy groups, which had a viscosity of 400 mPa·s, and 10 parts by weight of a fragrance material. An emulsion of the mixture was prepared by adding 3 parts by weight of polyoxyethylene lauryl ether (HLB=13.1 ) and 40 parts by weight of water and emulsifying. Subsequently, a solution of a 1,3-divinyltetramethyldisiloxane complex of platinum (platinum metal concentration: 0.04 wt%) was added to, and uniformly mixed with, the emulsion in an amount sufficient to produce a platinum metal concentration of 20 ppm based on the total weight of dimethylpolysiloxane, polyoxypropylene, and copolymer of dimethylsiloxane and methylhydrogensiloxane in the above-mentioned emulsion. Subsequently, the mixture was left to stand at room temperature for 1 day to carry out a hydrosilation reaction and prepare an emulsion of an organopolysiloxane composition. A test sample was prepared by combining 80 parts by weight of a hair conditioner (a mixture of 15 parts by weight of triethanolamine alkyl ether sulfate, 5 parts by weight of fatty acid monoethanolamide, 2 parts by weight of ethylene glycol monostearate, and 78 parts by weight of water) with 20 parts by weight of the emulsion. The fragrance retention properties, compounding stability, as well flexibility and smoothness of the sample were measured and the results are listed in Table 1. Also, a liquid with a viscosity of 2,000,000 mPa·s was obtained when water was removed from the emulsion by drying part of it at 105°C for 2 hours.

Application Example 2. A mixture was prepared comprising 1.2 parts by weight of a copolymer (content of silicon-bonded hydrogen atoms: 0.8 wt%) of dimethylsiloxane and methylhydrogensiloxane having both terminal ends of the molecular chain blocked by trimethylsiloxy groups, which had a viscosity of 15 mPa·s, 14.8 parts by weight of copolymer of oxypropylene and oxyethylene described by formula CH₂=CHCH₂(OC₂H₄)₁₀(OC₃H₆)₅₀ OH, which had one terminal end of the molecular chain blocked by an allyl group, and 7.5 parts by weight of dimethylpolysiloxane (content of vinyl groups: 0.5 wt%) having both terminal ends of the molecular chain blocked by dimethylvinylsiloxy groups, which had a viscosity of 400 mPa·s. An emulsion of the mixture was prepared by adding 1.5 parts by weight of polyoxyethylene lauryl ether (HLB=13.1) and 25 parts by weight of water and emulsifying. Subsequently, a solution of a 1,3-divinyltetramethyldisiloxane complex of platinum (platinum metal concentration: 0.04 wt%) was added to, and uniformly mixed with, the emulsion in an amount sufficient to produce a platinum metal concentration of 20 ppm based on the total weight of dimethylpolysiloxane, copolymer of oxypropylene and oxyethylene, and copolymer of dimethylsiloxane and methylhydrogensiloxane in the above-mentioned emulsion. Subsequently, the mixture was left to stand at room temperature for 1 day to carry out a hydrosilation reaction, whereupon a white uniform emulsion of an organopolysiloxane composition was prepared by slowly adding 50 parts by weight of a fragrance material in a dropwise manner to the mixture with agitation. A test sample was prepared by combining 80 parts by weight of a hair conditioner (a mixture of 15 parts by weight of triethanolamine alkyl ether sulfate, 5 parts by weight of fatty acid monoethanolamide, 2 parts by weight of ethylene glycol monostearate, and 78 parts by weight of water) with 20 parts by weight of the emulsion. The fragrance retention properties, compounding stability, as well flexibility and smoothness of the sample were measured and the results are listed in Table 1. Also, a liquid with a viscosity of 1,000,000 mPa·s was obtained when water was removed from the resultant emulsion by drying part of it at 105°C for 2 hours.

Comparative Example 1. An emulsion of an organopolysiloxane composition was prepared by mixing 42 parts by weight of dimethylpolysiloxane having both terminal ends of the molecular chain blocked by trimethylsiloxy groups, which had a viscosity of 1,000 mPa·s, with 5 parts by weight of a fragrance material, followed by adding 3 parts by weight of polyoxyethylene lauryl ether (HLB=13.1) and 50 parts by weight of water and emulsifying the mixture. A test sample was prepared by combining 80 parts by weight of a hair conditioner (a mixture of 15 parts by weight of triethanolamine alkyl ether sulfate, 5 parts by weight of fatty acid monoethanolamide, 2 parts by weight of ethylene glycol monostearate, and 78 parts by weight of water) with 20 parts by weight of the emulsion. The fragrance retention properties, compounding stability, as well flexibility and smoothness of the sample were measured and the results are listed in Table 1. Also, a liquid with a viscosity of 900 mPa·s was obtained when water was removed from the emulsion by drying part of it at 105°C for 2 hours.

Comparative Example 2. A mixture was formed comprising 1.7 parts by weight of copolymer (content of silicon-bonded hydrogen atoms: 0.1 wt%) of dimethylsiloxane and methylhydrogensiloxane having both terminal ends of the molecular chain blocked by trimethylsiloxy groups, which had a viscosity of 15 mPa·s, 40 parts by weight of copolymer of dimethylsiloxane and methylvinylsiloxane (content of vinyl groups: 0.5 wt%) having both terminal ends of the molecular chain blocked by dimethylvinylsiloxy groups, which had a viscosity of 400 mPa·s, 5 parts by weight of a fragrance material, and a solution of a 1,3-divinyltetramethyldisiloxane complex of platinum, in an amount sufficient to produce a platinum metal concentration of 20 ppm based on the total weight of the above-mentioned copolymer of dimethylsiloxane and methylhydrogensiloxane and copolymer of dimethylsiloxane and methylvinylsiloxane. Subsequently, an emulsion of the mixture was prepared by adding 3 parts by weight of polyoxyethylene cetyl ether (HLB=17.0) and 50 parts by weight of water and emulsifying. An emulsion of an organopolysiloxane composition was then obtained by allowing the emulsion to stand at room temperature for 1 day to carry out a hydrosilation reaction. A test sample was prepared by combining 80 parts by weight of a hair conditioner (a mixture of 15 parts by weight of triethanolamine alkyl ether sulfate, 5 parts by weight of fatty acid monoethanolamide, 2 parts by weight of ethylene glycol monostearate, and 78 parts by weight of water) with 20 parts by weight of the emulsion. The fragrance retention properties, compounding stability, as well flexibility and smoothness of the sample were measured and the results are listed in Table 1. Also, a liquid with a viscosity of 1,000,000 mPa·s was obtained when water was removed from the resultant emulsion by drying part of it at 105°C for 2 hours.

**Table 1**

| Examples Evaluation parameters | Application Example 1 | Application Example 2 | Comparative Example 1 | Comparative Example 2 |
|---|---|---|---|---|
| Fragrance retention properties 1 day later | A | A | B | A |
| 2 days later | A | A | C | C |
| 3 days later | A | A | C | C |
| Compounding stability | A | A | C | B |
| Flexibility | B | A | D | C |
| Smoothness | A | A | C | A |

## Claims

1. An organopolysiloxane composition comprising
(A) 5 to 99.9 wt% of a crosslinked product of organopolysiloxane which is liquid or gel-like at normal temperature, the crosslinked product containing silicon-bonded polyoxyalkylene groups described by general formula―R¹(OR²)ₘOR³ , where R¹ and R² are independently selected alkylene groups, R³ is selected from the group consisting of a hydrogen atom, alkyl groups, acyl groups, and isocyan group, and *m* is a positive integer, and
(B) 95 to 0.1 wt% of a fragrance material.

2. The organopolysiloxane composition of claim 1, where component (A) is a crosslinked product obtained by a hydrosilation reaction between
(a) an organopolysiloxane having at least two silicon-bonded hydrogen atoms in each molecule,
(b) a compound selected from (b-1) a polyoxyalkylene described
by formula R⁴(OR²)ₘOR³, where R², R³, and *m* are the same as above, and R⁴ is an alkenyl group and (b-2) an organopolysiloxane having silicon-bonded polyoxyalkylene groups described by formula―R¹(OR²)ₘOR³, where R', R², R³, and *m* are the same as above, and silicon-bonded alkenyl groups or hydrogen atoms, and
(c) an organopolysiloxane having at least two silicon-bonded alkenyl groups in each molecule.

3. The organopolysiloxane composition of claim 1 or 2, where component (A) is a crosslinked product obtained by a hydrosilation reaction between
(a) 1 part by weight of an organopolysiloxane having at least two silicon-bonded hydrogen atoms in each molecule,
(b) 0.1 to 100 parts by weight of a compound selected from (b-1)
a polyoxyalkylene described by formula R⁴(OR²)ₘOR³, where R², R³, and *m* are the same as above, and R⁴ is an alkenyl group and (b-2) an organopolysiloxane having silicon-bonded polyoxyalkylene groups described by formula ―R¹(OR²)ₘOR³, where R¹, R², R³, and *m* are the same as above, and silicon-bonded alkenyl groups or hydrogen atoms,
(c) 0.01 to 100 parts by weight of an organopolysiloxane having at least two silicon-bonded alkenyl groups in each molecule, and
(d) a catalytic amount of a hydrosilylation catalyst.

4. An organopolysiloxane composition according to any of claims 1 to 3, where subscript *m* is in the range of from 20 to 80.

5. An organopolysiloxane composition according to any of claims 1 to 4, where component (A) has a viscosity at 25°C in the range of from 100,000 mPa·s to 10,000,000 mPa·s.

6. An organopolysiloxane composition according to any of claims 1 to 5, where the proportion of component (A) to component (B) is in the range of 40 to 99 : 60 : 1 wt %.

7. The organopolysiloxane composition of claim 2 or 3, where component (b-1) is a polyoxyalkylene described by formula R⁴(OC₂H₄)ₚ(OC₃H₆)_{q}OR³, where R³ and R⁴ are the same as above, *p* is 0 or a positive integer, and *q* is a positive integer, with the proviso that *p*<*q*.

8. The organopolysiloxane composition according to any preceding claim emulsified in water.

9. A method for preparing an organopolysiloxane composition comprising crosslinking and aqueous emulsion comprising (A) 5 to 99.9 wt% of a crosslinkable organopolysiloxane composition comprising
(a) 1 part by weight of an organopolysiloxane having at least two silicon-bonded hydrogen atoms in each molecule,
(b) 0.1 to 100 parts by weight of a compound selected from (b-1)
a polyoxyalkylene described by formula R⁴(OR²)ₘOR³, where R² is an alkylene group, R³ is selected from the group consisting of a hydrogen atom, alkyl groups, acyl groups, and isocyano group, R⁴ is an alkenyl group, and *m* is a positive integer and (b-2) an organopolysiloxane having silicon-bonded polyoxyalkylene groups described by formula ―R¹(OR²)ₘOR³, where R¹ is an alkylene group, R², R³, and *m* are the same as above, and silicon-bonded alkenyl groups or hydrogen atoms,
(c) 0.01 to 100 parts by weight of an organopolysiloxane having at least two silicon-bonded alkenyl groups in each molecule, and
(d) a catalytic amount of a hydrosilylation catalyst; and (B) 95 to 0.1 wt% of a fragrance material.

10. The method for preparing an organopolysiloxane composition according to claim 9, where the crosslinking is carried out by emulsifying components (a) to (c) and the fragrance material (B) in water and then adding component (d).

11. The method for preparing an organopolysiloxane composition according to claim 9 or 10, where component (b-1) is a polyoxyalkylene described by formula R⁴(OC₂H₄)ₚ(OC₃H₆)_{q}OR³ , where R³ and R⁴ are as above, *p* is 0 or a positive integer and *q* is a positive integer, with the proviso the *p<q*.

12. A method for preparing an organopolysiloxane composition comprising crosslinking and aqueous emulsion comprising (A) 5 to 99.9 wt% of a crosslinkable organopolysiloxane composition comprising
(a) 1 part by weight of an organopolysiloxane having at least two silicon-bonded hydrogen atoms in each molecule,
(b) 0.1 to 100 parts by weight of a compound selected from (b-1)
a polyoxyalkylene described by formula R⁴(OR²)ₘOR³, where R² is an alkylene group, R³ is selected from the group consisting of a hydrogen atom, alkyl groups, acyl groups, and isocyano group, R⁴ is an alkenyl group, and *m* is a positive integer and (b-2) an organopolysiloxane having silicon-bonded polyoxyalkylene groups described by formula ―R¹(OR²)ₘOR³, where R¹ is an alkylene group, R², R³, and *m* are the same as above, and silicon-bonded alkenyl groups or hydrogen atoms,
(c) 0.01 to 100 parts by weight of an organopolysiloxane having at least two silicon-bonded alkenyl groups in each molecule, and
(d) a catalytic amount of a hydrosilylation catalyst; and adding (B) 95 to 0.1 wt% of a fragrance material to the emulsified crosslinked product.

13. The method for preparing an organopolysiloxane composition according to claim 12, where components (a) to (c) are emulsified in water and then component (d) is added to effect crosslinking of components (a) to (c), followed by adding fragrance material (B).

14. The method for preparing an organopolysiloxane composition according to claim 12 or 13, where component (b-1) is a polyoxyalkylene described by formula
R⁴(OC₂H₄)ₚ(OC₃H₆)_{q}OR³ , where R³ and R⁴ are as described above, *p* is 0 or a positive integer and *q* is a positive integer, with the proviso that *p<q*.

15. An organopolysiloxane composition comprising
(A) 5 to 99.9 wt% of a crosslinked product of organopolysiloxane which is liquid or gel-like at normal temperature obtained by a hydrosilation reaction between
(a) an organopolysiloxane having at least two silicon-bonded hydrogen atoms in each molecule,
(b) a compound selected from the group consisting of (b-1) a polyoxyalkylene described by formula R⁴(OR²)ₘOR³ , where R² is an alkylene group, R³ is selected from the group consisting of a hydrogen atom, alkyl groups, acyl groups, and isocyan group, R⁴ is an alkenyl group and *m* is a positive integer, and, (b-2) an organopolysiloxane having silicon-bonded polyoxyalkylene groups described by formula -R¹(OR²)ₘOR³, where R² is an alkylene group, R³ is selected from the group consisting of a hydrogen atom, alkyl groups, acyl groups, and isocyan group, and *m* is a positive integer, and
(c) an organopolysiloxane having at least two silicon-bonded alkenyl groups in each molecule; and (B) 95 to 0.1 wt% of a fragrance material.
